# EUROPEAN PATENT SPECIFICATION

(11) **EP 3 528 891 B1**
(45) Date of publication and mention of the grant of the patent: **03.12.2025**
(21) Application number: 17862609.9
(22) Date of filing: 23.10.2017
(51) Int. Cl.: A61N 5/06, A61N 5/067, A61N 5/08, A61K 41/00

(54) **SYSTEMS AND METHODS FOR THERMAL GRADIENT PRECONDITIONING FOR SELECTIVE PHOTOTHERMAL TARGETING**
SYSTEME UND VERFAHREN ZUR VORKONDITIONIERUNG VON WÄRMEGRADIENTEN FÜR SELEKTIVES FOTOTHERMISCHES TARGETING
SYSTÈMES ET PROCÉDÉS DE PRÉCONDITIONNEMENT À GRADIENT THERMIQUE POUR CIBLAGE PHOTOTHERMIQUE SÉLECTIF

(30) Priority: 21.10.2016 US 201662411149 P
(43) Date of publication of application: 28.08.2019
(73) Proprietor: The General Hospital Corporation, Boston, MA 02114 (US)
(72) Inventor: SAKAMOTO, Fernanda, H., Boston, MA 02114 (US); ANDERSON, R., Rox, Boston, MA 02114 (US); FARINELLI, William, A., Danvers, MA 01923 (US)
(74) Representative: Maiwald GmbH
(86) International application number: PCT/US2017/057893
(87) International publication number: WO 2018/076011

(56) References cited:
- EP-A2- 0 991 372
- EP-B1- 0 991 372
- WO-A1-2015/117005
- US-A1- 2006 009 750
- US-A1- 2010 174 223
- US-A1- 2011 160 712
- C STURESSON ET AL: "Mathematical modelling of dynamic cooling and pre-heating, used to increase the depth of selective damage to blood vessels in laser treatment of port wine stains", PHYSICS IN MEDICINE AND BIOLOGY, vol. 41, no. 3, 1 March 1996 (1996-03-01), Bristol GB, pages 413 - 428, XP055682556, ISSN: 0031-9155, DOI: 10.1088/0031-9155/41/3/006
- SAKAMOTO, F. ET AL.: "Selective Photothermolysis to Target Sebaceous Glands: Theoretical Estimation of Parameters and Preliminary Results Using a Free Electron Laser", LASERS SURG. MED., vol. 44, 13 December 2011 (2011-12-13), pages 175 - 183, XP055478280, Retrieved from the Internet <URL:https://www.osti.gov/scitech/servlets/purl/1039896> [retrieved on 20171130]

## Description

### BACKGROUND OF THE INVENTION

The present disclosure generally relates to improvements to systems and methods for targeting specific chromophores embedded in a medium. Specifically, the present disclosure relates to systems and methods for targeting sebaceous glands and initiating damage in the sebaceous glands without exceeding a given damage threshold for adjacent tissues.

The concept of targeting sebaceous glands using optical excitation of sebum to initiate thermal damage is known in the art. However, in order to achieve the thermal damage, a significant amount of light must be applied, which is absorbed both by the sebaceous glands, and by higher layers of skin and results in a significant increase in temperature. This increased temperature causes pain in the subject, and can cause unwanted damage to the epidermis overlying the target glands.

In order to counteract the pain, various attempts have been made to apply surface cooling. However, surface cooling is insufficient to overcome the pain induced by thermal damage for some chromophores, such as sebum. In the case of sebum and inducing damage to the sebaceous gland, surface cooling has not been able to prevent pain.

There exists a clear need for improvements that allow for inducing thermal damage in various chromophores, such as sebum, while staying below a pain and unwanted damage threshold for the subject.

US 2011/160712 A1 describes a laser treatment system for producing thermal cavities and energy droplets.

### SUMMARY OF THE INVENTION

The present invention overcomes drawbacks of previous technologies by providing a system and a cosmetic method for providing a controlled thermal treatment to a target. The invention is defined in the appended claims.

### SUMMARY OF THE DISCLOSURE

In an aspect, the present disclosure provides a method of providing a controlled thermal treatment to a target medium. The method can include one or more of the following steps: preconditioning the target medium to establish a thermal gradient having a peak temperature at a predefined depth from a surface of the target medium; and phototreating the target medium.

In another aspect, the present disclosure provides a system. The system can include a preconditioning subsystem, a phototreating subsystem, a processor, and a memory having stored thereon computer executable instructions that, when executed by the processor, cause the processor to perform a method as described herein, the processor configured to execute the computer-executable instructions.

The foregoing and other advantages of the disclosure will appear from the following description. In the description, reference is made to the accompanying drawings which form a part hereof, and in which there is shown by way of illustration certain aspects of the disclosure. These aspects do not necessarily represent the full scope of the disclosure, however, and reference is made therefore to the claims and herein for interpreting the scope of the disclosure.

### BRIEF DESCRIPTION OF THE DRAWINGS

The present disclosure will hereafter be described with reference to the accompanying drawings, wherein like reference numerals denote like elements.
Fig. 1 is a schematic of a system, in accordance with an aspect of the present disclosure.
Fig. 2 is a schematic of a system, in accordance with an aspect of the present disclosure.
Fig. 3 is a plot showing a thermal gradient, in accordance with an aspect of the present disclosure.
Fig. 4 is a schematic of a scanning regimen, in accordance with an aspect of the present disclosure.
Fig. 5 is a flowchart illustrating a method, in accordance with an aspect of the present disclosure.
Fig. 6 is a flowchart illustrating a method, in accordance with an aspect of the present disclosure.

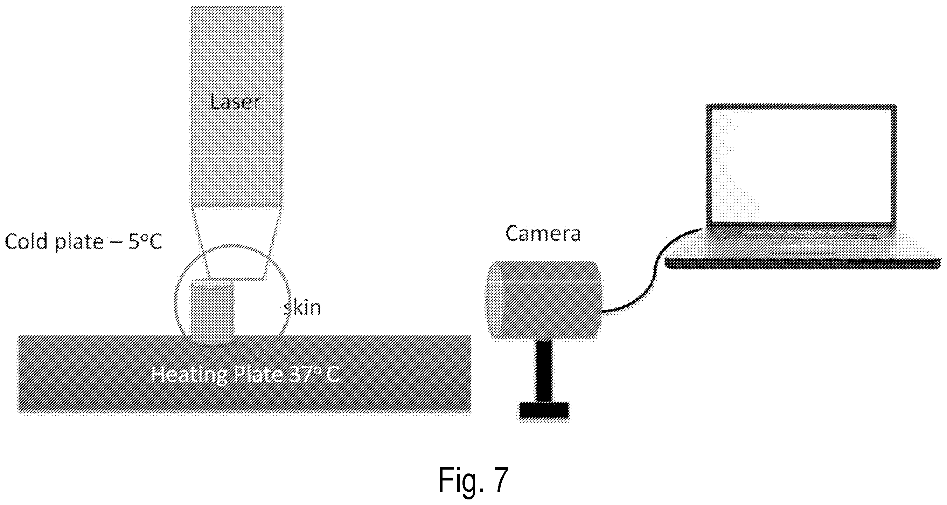

Fig. 7 is a schematic of an experimental setup utilized in Example 12 and elsewhere.

### DETAILED DESCRIPTION

Before the present invention is described in further detail, it is to be understood that the invention is not limited to the particular embodiments described. It is also to be understood that the terminology used herein is for the purpose of describing particular embodiments only, and is not intended to be limiting. The scope of the present invention will be limited only by the claims. As used herein, the singular forms "a", "an", and "the" include plural embodiments unless the context clearly dictates otherwise.

It should be apparent to those skilled in the art that many additional modifications beside those already described are possible without departing from the scope of the present invention as defined by the appended claims. In interpreting this disclosure, all terms should be interpreted in the broadest possible manner consistent with the context. Variations of the term "comprising", "including", or "having" should be interpreted as referring to elements, components, or steps in a non-exclusive manner, so the referenced elements, components, or steps may be combined with other elements, components, or steps that are not expressly referenced. Embodiments referenced as "comprising", "including", or "having" certain elements are also contemplated as "consisting essentially of" and "consisting of" those elements, unless the context clearly dictates otherwise. It should be appreciated that aspects of the disclosure that are described with respect to a system are applicable to the methods, and vice versa, unless the context explicitly dictates otherwise.

Numeric ranges disclosed herein are inclusive of their endpoints. For example, a numeric range of between 1 and 10 includes the values 1 and 10. When a series of numeric ranges are disclosed for a given value, the present disclosure expressly contemplates ranges including all combinations of the upper and lower bounds of those ranges. For example, a numeric range of between 1 and 10 or between 2 and 9 is intended to include the numeric ranges of between 1 and 9 and between 2 and 10.

This disclosure provides systems and methods for providing a controlled thermal treatment to a target. As discussed above, existing systems and methods have applied thermal treatment by way of electromagnetic radiation, which in some cases has a selective absorption in a chromophore of interest. In addition, surface cooling has been applied to help reduce the amount of pain introduced by non-selective absorption of the electromagnetic radiation. However, neither of these approaches has yielded a treatment that effectively damages the chromophore or material surrounding the chromophore while remaining below a tolerable pain threshold for the patient. The present disclosure provides systems and methods that can involve a preconditioning that utilizes surface cooling in combination with a preheating (for example, a light-induced preheating) to establish a thermal gradient that has a peak temperature at a depth where the target chromophore of interest is located and/or expected to be located. A subsequent phototreatment can then be introduced to raise the temperature of the chromophore to a level where target damage is induced, while the temperature of tissue above and below the depth of the chromophore remains low enough that the subject does not suffer unwanted damage and/or experience extreme pain. The degree of selectivity in introducing damage and the extent to which the pain to the subject is reduced by the systems and methods described herein is surprising and marks a substantial improvement to the present state of the art.

### Systems

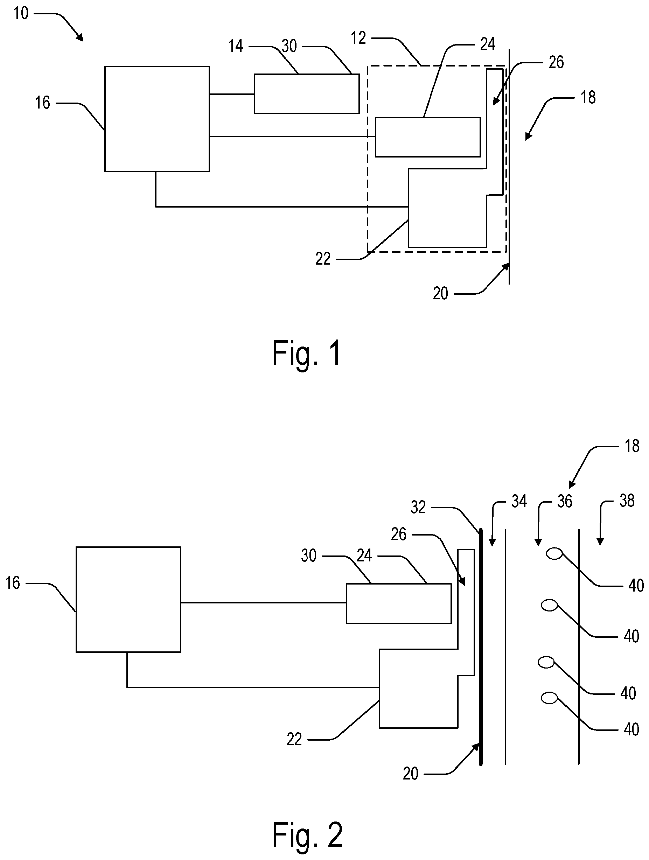

Referring to Fig. 1, the present disclosure provides a system 10. The system 10 can include a preconditioning subsystem 12, a phototreatment system 14, and a computer 16. As discussed in greater detail below, the system 10 is configured to provide treatment to a target medium 18 having a target surface 20.

The preconditioning subsystem 12 can include a preconditioning cooling device 22 and a preconditioning heating device 24.

The preconditioning cooling device 22 can be a device that a person having ordinary skill in the art would recognize as suitable for applying cooling to the target medium 18. In certain aspects, the preconditioning cooling device 22 can be a conductive or convective cooling device. Example of suitable preconditioning cooling devices 22 include, but are not limited to, a thermally conductive material (for example, metal, glass, ceramic, a sapphire window, a gaseous fluid such as air, a liquid fluid such as heavy water, a pulsed spray of liquid or gaseous fluid, and the like) coupled to a cooling circuit. One specific non-limiting example of a suitable preconditioning cooling device 22 includes two windows (such as two sapphire windows) having a fluid in between, such as a gaseous or liquid fluid, where the fluid is circulated by a cooling circuit. The cooling circuit can be constructed according to the knowledge of a person having ordinary skill in the thermal circuitry arts. In certain aspects, the preconditioning cooling device 22 can include a surface contact portion 26 configured to contact the target surface 20 for the application of cooling. In certain aspects, the surface contact portion 24 can be transparent to various portions of the electromagnetic spectrum. In certain aspects, the surface contact portion 26 can include one or more apertures configured to provide vias for transmitting one or more sources of heat from the preconditioning heating device 24 (for example, the apertures can allow electrodes, ultrasound energy, electromagnetic radiation, or the like to pass).

The preconditioning heating device 24 can be a radiofrequency source including one or more electrodes, an ultrasound probe configured to provide a thermal effect to the target medium 18, a preconditioning microwave source, or a preconditioning light source.

In certain aspects, the preconditioning heating device 24 can be a preconditioning light source. The preconditioning light source can be a laser (for example, a fiber laser, a diode laser, or other suitable laser), a light-emitting diode, an incandescent light source, an arc lamp (also known as a discharge lamp), a flash lamp (for example, a Xe flash lamp), or the like.

The preconditioning subsystem 12 can include various electronics, power supplies, controls, circuitry, optics, or the like in order to provide suitable function in accordance with the performance described herein.

The phototreatment subsystem 14 can include a phototreatment light source 30. The phototreatment light source 30 can be a laser (for example, a fiber laser, a diode laser, or other suitable laser), a flash lamp (for example, a Xe flash lamp), or the like.

The phototreatment subsystem 14 can include various electronics, power supplies, controls, circuitry, optics, or the like in order to provide suitable function in accordance with the performance described herein.

In certain aspects, the preconditioning light source and the phototreatment light source 30 can be the same device. When the preconditioning light source and the phototreatment light source 30 are the same device, that device can be a laser (for example, a fiber laser, a diode laser, or other suitable laser), a flash lamp (for example, a Xe flash lamp), or the like.

The preconditioning light source and/or the phototreatment light source 30 can be configured to emit light having a wavelength or a wavelength range that penetrates to a desired depth into a target medium 18, including a wavelength or a wavelength range of between 350 nm and 2000 nm, including but not limited to, a wavelength or a wavelength range of between 600 nm and 1100 nm, between 610 nm and 700 nm, between 1600 nm and 1800 nm, between 1000 nm and 1500 nm, between 450 nm and 650 nm, or other wavelengths or wavelength ranges suitable for achieving the desired effects described herein. In certain aspects, the preconditioning light source and/or the phototreatment light source 30 can be configured to emit light having a wavelength tuned to an absorption peak of a chromophore of interest. In certain aspects, the preconditioning light source and/or the phototreatment light source 30 can be configured to emit light having a wavelength of 760 nm, 920 nm, 1064 nm, 1210 nm, 1726 nm, or other wavelengths suitable for achieving the desired effects described herein. It should be appreciated that where a wavelength is defined, a nominal wavelength for an absorption band is also contemplated.

A person having ordinary skill in the art would appreciate that selection of the wavelength can involve a host of variables, including the location, intensity, and shape of an absorption band for a target chromophore, as well as the location, intensity, and shape of absorption bands for competing chromophores. Thus, in some cases, the wavelength might be selected for a peak absorption for a target chromophore, so long as competing chromophores do not significantly interfere with absorption at that peak absorption wavelength. In other cases, the wavelength might be selected for an absorption that remains significant but is weaker than the peak absorption, but where competing chromophores might interfere at the wavelength of peak absorption and do not interfere at the selected wavelength. In the case of preconditioning, the wavelength is selected to suitably achieve a spatial temperature gradient as described herein, and the preconditioning effects described herein. In the case of phototreatment, the wavelength is selected to suitably achieve the phototreatment effects described herein.

The preconditioning light source and/or the phototreatment light source 30 can be configured to emit continuous wave light, pulses of light, or both. The preconditioning light source and/or the phototreatment light source 30 can be configured to be switchable between continuous wave light and pulses of light. In certain circumstances, the preconditioning light source and/or the phototreatment light source 30 can have a shaped beam, where the emitted energy is larger in diameter at the surface and more focused at a desired depth. One means of accomplishing this would be through the use of a high numerical aperture objective. In these circumstances, it may be beneficial to scan the shaped beam, for example by using the methods described elsewhere herein.

The preconditioning light source and/or the phototreatment light source 30 can be configured to emit pulses of light having a pulse width of between 10 fs and continuous wave, including but not limited to, a pulse width of between 100 fs and 1 second, between 1 ps and 750 ms, between 10 ps and 500 ms, between 100 ps and 250 ms, between 1 ns and 100 ms, between 10 ns and 50 ms, between 100 ns and 10 ms, between 1 µs and 750 ms, between 10 µs and 500 ms, between 50 µs and 250 ms, between 100 µs and 100 ms, between 500 µs and 50 ms, or between 1 ms and 25 ms. The depth of penetration into tissue of the preconditioning source is such that a tissue layer containing subsurface targets can be warmed prior to arrival of a treatment pulse. The absorbed average power density of the preconditioning source is such that cooling at the tissue surface maintains a substantially lower, non-damaging temperature than that at the target tissue layer, prior to arrival of a treatment pulse. Pulses for either the preconditioning or treatment sources, may be provided by a pulsed source, such as a pulsed laser, or by spatially scanning a continuous or quasicontinuous source to achieve the desired average absorbed power density. The pulse width as described herein is measured as a full-width at half maximum using methods known to those having ordinary skill in the art. The preconditioning light source and/or the phototreatment light source 30 can be configured to emit pulses of light having pulse separation (measured as a peak-to-peak separation of pulses by methods known to those having ordinary skill in the art) of between 100 fs and 1 second, including but not limited to, a pulse separation of between 1 ps and 750 ms, between 10 ps and 500 ms, between 100 ps and 250 ms, between 1 ns and 100 ms, between 10 ns and 50 ms, between 100 ns and 10 ms, between 1 µs and 750 ms, between 10 µs and 500 ms, between 50 µs and 250 ms, between 100 µs and 100 ms, between 500 µs and 50 ms, or between 1 ms and 25 ms. A person having ordinary skill in the art should appreciate that the use of a pulsed source for preconditioning and phototreatment remains within the confines of the desired effect of the preconditioning and phototreatment. For example, a pulsed source for preconditioning can be configured to provide a desired average power density to establish a thermal gradient as described elsewhere herein without otherwise causing a thermal consequence (such as the phototreatment). As another example, a pulse source for phototreatment can provide the desired thermal consequence following the mechanism of selective photothemolysis using one pulse (or more in certain cases) of light.

In certain aspects, the phototreatment light source 30 can be configured to emit pulses of light having a pulse width of between 1 µs and 750 ms, including but not limited to, a pulse width of between 10 µs and 500 ms, between 50 µs and 250 ms, between 100 µs and 100 ms, between 500 µs and 50 ms, or between 1 ms and 25 ms.

The computer 16 can take the form of a general purpose computer, a tablet, a smart phone, or other computing devices that can be configured to control the devices described herein, and which can execute a computer executable program that performs the methods described herein. The computer 24 can include various components known to a person having ordinary skill in the art, such as a processor and/or a CPU, memory of various types, interfaces, and the like. The computer 24 can be a single computing device or can be a plurality of computing devices operating in a coordinated fashion.

The processor and/or CPU can be configured to read and perform computer-executable instructions stored in the memory. The computer-executable instructions can include all or portions of the methods described herein.

The memory can include one or more computer readable and/or writable media, and may include, for example, a magnetic disc (e.g., a hard disk), an optical disc (e.g., a DVD, a Blu-ray, a CD), a magneto-optical disk, semiconductor memory (e.g., a non-volatile memory card, flash memory, a solid state drive, SRAM, DRAM), an EPROM, an EEPROM, and the like. The memory can store the computer-executable instructions for all or portions of the methods described herein.

The interfaces can provide communication interfaces to input and output devices, which can include a keyboard, a display, a mouse, a printing device, a touch screen, a light pen, an optical storage device, a scanner, a microphone, a camera, a drive, a communication cable, or a network (wired or wireless). The interfaces can also provide communications interfaces to the other components included in the system 10 and/or used in the methods described herein.

The system 10 can include one or more power supplies (not illustrated) for providing power to the various components of the system 10.

Referring to Fig. 2, one example of a system 10 is illustrated as deployed in a specific arrangement, namely where the preconditioning heating device 26 and the phototreatment light source 30 are a single light source, and in a specific context, namely for the treatment of sebaceous glands.

In the specific context of Fig. 2, the target medium 18 can be a layered system, such as human skin. The target medium 18 can include a first layer or a stratum corneum layer 32, a second layer or an epidermis layer 34, a third layer or a dermis layer 36, and a fourth layer or a subcutaneous layer 38. The dermis layer 36 can contain a plurality of target chromophores or sebaceous glands 40. It should be appreciated that target chromophores 40 are not limited to the third layer or dermis layer 36 and can be located in various other layers and/or at various different depths.

The system 10 can include a light translation element (not illustrated) for laterally translating light emitted from one or more of the light sources described herein. The light translation element can be a 1-, 2-, or 3-dimensional translation stage that the light source is mounted to, whereby the entire light source is moved in order to translate the emitted light. The light translation element can also be an optical setup that allows the light itself to be translated without requiring the movement of the light source. A person having ordinary skill in the art would recognize suitable light translation elements including, but not limited to, a single mirror, a pair of mirrors, one or more mirrors coupled to a motor that changes the angle and/or position of emitted light, and the like.

In certain aspects, the target chromophore 40 can be sebum, melanin, hemoglobin, oxyhemoglobin, reduced hemoglobin, water, and the like. In certain aspects, the target chromophore 40 is sebum.

Aspects of the disclosure described elsewhere herein with respect to the methods are applicable to the system 10, unless the context clearly dictates otherwise. For example, if a given wavelength or pulse width is described with respect to a method, then the system 10 should be interpreted as providing that given wavelength or pulse width.

### Methods

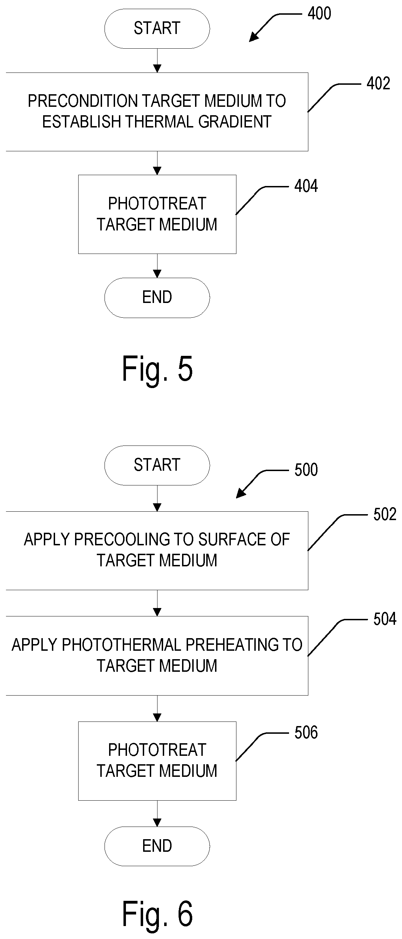

This disclosure also provides methods for thermal gradient preconditioning for selective targeting. In certain aspects, referring to Fig. 5, the method 400 can include one or more the following steps: at process block 402, preconditioning a target medium to establish a thermal gradient; and at process block 404, phototreating the target medium. The thermal gradient can have a peak temperature at a predefined depth from a surface of the target medium. The preconditioning can include applying a conductive or convective cooling to a surface of the target medium and applying a propagating heating energy into the target medium via the surface of the target medium.

In certain aspects, referring to Fig. 6, the method 500 can include one or more of the following steps: at process block 502, applying a precooling to a surface of the target medium; at process block 504, applying a photothermal preheating to the target medium; and at process block 506, phototreating the target medium.

In certain aspects, preconditioning a target medium can include simultaneously applying a precooling to a surface of the target medium and applying a photothermal preheating to the target medium.

The methods described herein can be useful for the treatment of various clinical indications, including but not limited to, acne, benign cutaneous tumors (e.g., xanthoma/xanthelasma, syringomas), granulomas, subcutaneous fat and cellulite, angiokeratoma, ganglion cysts, cutaneous calcinosis, and other conditions of the like. In certain cases, the methods described herein can be useful for the treatment of acne.

The methods described herein are configured to provide treatment to a subject that induces pain in the subject that is less than a pre-defined pain threshold for the subject. There are a variety of ways of determining a pain level in a subject, as known in the art (for example, a 1 to 10 point scale, etc.). The pre-defined pain threshold can be defined based on the context, such that a higher pain threshold might be used for an urgent treatment or a lower pain threshold might be used for a purely cosmetic treatment. Calibrating the treatment to a given user can be achieved by establishing a pain threshold calibration plot for the given user via testing a series of known treatments on a user. Calibrating the treatment to a given user can utilize real-time feedback on pain from the subject. The pain threshold can be determined for a user in the presence or absence of pain medication. A statistical distribution of pain thresholds can be determined for a chosen group of subjects, and the methods can be configured to be less than a given percentile pain threshold for the chosen group, such as less than the 99th percentile pain threshold, less than the 95th percentile pain threshold, less than the 90th percentile pain threshold, less than the 80th percentile pain threshold, or less than the 75th percentile pain threshold. In certain aspects, the pain threshold for preconditioning can be the same as or different than the pain threshold for phototreating.

The applying a precooling to the surface or the applying a conductive or convective cooling to a surface of the target medium can include contacting the surface of the target medium with a temperature controlled device, such as the surface contact portion 26 of the preconditioning cooling device.

In certain aspects, the applying a precooling to the surface can include applying the precooling for a length of time of between 1 s and 5 minutes, including but not limited to, a length of time of between 2 s and 2 minutes, between 3 s and 1 minute, between 4 s and 50 s, between 5 s and 45 s, between 6 s and 40 s, between 7 s and 30 s, between 8 s and 25 s, between 9 s and 20 s, between 10 s and 15 s, or between 15 s and 30 s. A person having ordinary skill in the art will recognize that the length of time of applying the precooling can be dependent on a number of factors, such as the desired depth of penetration of the precooling.

In certain aspects involving contacting the surface of the target medium with a temperature controlled device, the temperature controlled device can have a temperature between -10 °C and 20 °C, including but not limited to a temperature between -5 °C and 10 °C, between -2.5 °C and 5 °C, or between 0 °C and 2.5 °C. In certain aspects, the applying the precooling to the surface can include extracting thermal power in an amount of between 0.1 W/cm² and 10 W/cm², including but not limited to, an amount of between 0.5 W/cm² and 9.0 W/cm², between 1 W/cm² and 8.0 W/cm², between 2.5 W/cm² and 7.5 W/cm², or between 4 W/cm² and 6 W/cm².

The applying a propagating heating energy into the target medium via the surface of the target medium can include applying radiofrequency energy into the target medium via the surface of the target medium, applying ultrasound energy into the target medium via the surface of the target medium, applying electromagnetic radiation into the target medium via the surface of the target medium, or a combination thereof.

The applying electromagnetic radiation into the target medium or applying a photothermal preheating to the target medium can include transmitting light have a preheating absorbed average power density or absorbed average irradiance for a preheating length of time. In certain aspects, the preheating absorbed average power density or absorbed average irradiance can be less than a phototreating absorbed average power density or absorbed average irradiance of light used in the phototreating and/or the preheating length of time can be less than a phototreating length of time for which the phototreating is applied.

In certain aspects, the preheating absorbed average power density or absorbed average irradiance can be between 0.1 W/cm² and 10 W/cm², including but not limited to, a preheating absorbed average power density or absorbed average irradiance of between 0.25 W/cm² and 9 W/cm², between 0.5 W/cm² and 7.5 W/cm², between 0.75 W/cm² and 5 W/cm², between 1 W/cm² and 4 W/cm², or between 2 W/cm² and 3 W/cm². In certain aspects, the preheating length of time can be between 1 s and 5 minutes, including but not limited to, a length of time of between 2 s and 2 minutes, between 3 s and 1 minute, between 4 s and 50 s, between 5 s and 45 s, between 6 s and 40 s, between 7 s and 30 s, between 8 s and 25 s, between 9 s and 20 s, between 10 s and 15 s, or between 15 s and 30 s.. The absorbed average irradiance, as used herein and assuming complete absorption, refers to the irradiance of a light source times the quantity 1 minus the reflectance.

In certain aspects, the applying a precooling to a surface of the target medium and the applying a photothermal preheating to the target medium steps can be simultaneous or sequential. In simultaneous aspects, the applying a precooling and the applying a photothermal preheating can completely overlap in time or can overlap in time for a portion of either applying step. As an example of complete overlap, the applying the precooling and the applying the preheating can both be applied together for a period of 20 seconds. As an example of partial overlap, the applying the precooling can be applied alone for a period of 10 seconds and the applying the precooling and the applying the preheating can both be applied together for a period of 10 seconds. It should be appreciated that a simultaneous or sequential application of precooling and/or photothermal preheating that achieves the desired thermal gradient is contemplated without limitation.

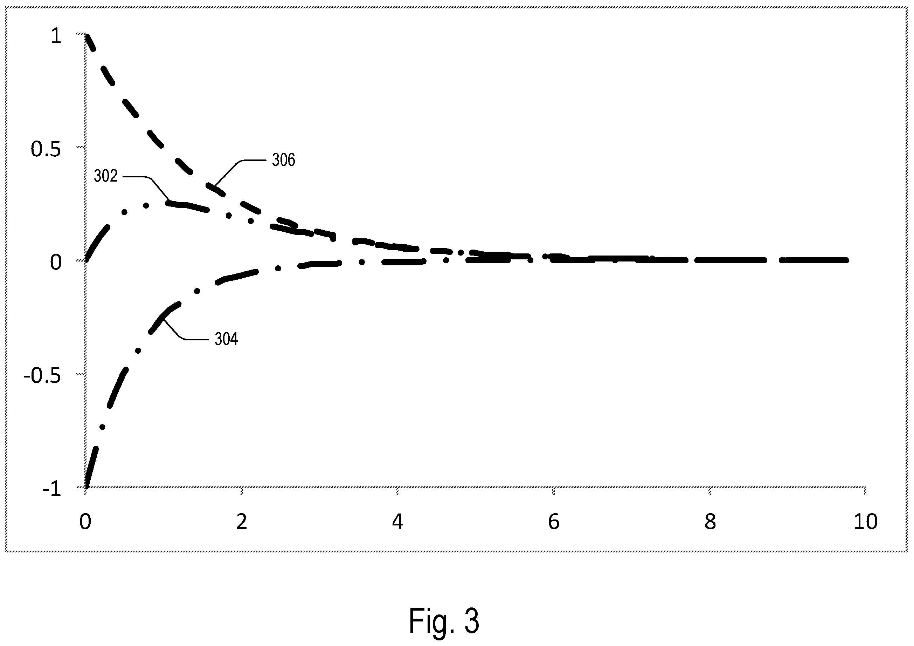

As discussed elsewhere, the thermal gradient can be established by combining a cooling component of the thermal gradient (for example, the portion of the thermal gradient generated by the precooling) and a heating component of the thermal gradient (for example, the portion of the thermal gradient generated by the preheating). Referring to Fig. 3, an example thermal gradient 302 is plotted. The units are arbitrary and the plot is intended to represent the concept generally without limiting the ways in which the thermal gradient can be applied. The thermal gradient 302 is a combination of the cooling component 304 and the heating component 306. In the illustrated example, the cooling component 304 and the heating component 306 have the same maximum magnitude, but it should be appreciated that either the cooling component 304 or the heating component 306 can have a larger absolute magnitude. In the illustrated example, the cooling component 304 and the heating component 306 are exponentially decaying functions. The cooling component 304 and/or the heating component 306 can take on various functional forms, depending on the absorption and conduction properties of the target medium. In the illustrated example, the cooling component 304 has a decay rate that is twice the decay rate of the heating component 306. It should be appreciated that the decay rate of the cooling component 304 and the heating component 306 can be the same or different, and either can be larger or smaller than the other. A person having ordinary skill in the art will recognize how to combine various cooling components 304 and heating components 306 to achieve a thermal gradient 302 with a maximum temperature at a desired depth. A person having ordinary skill in the art will also recognize that, due to scattering, the components of the thermal gradient may not be exponential as shown in Fig. 3.

In certain aspects, the desired depth for the maximum temperature of the thermal gradient 302 can be a depth between 1 µm and 100 mm, including but not limited to, a depth between 10 µm and 75 mm, between 50 µm and 50 mm, between 100 µm and 40 mm, between 250 µm and 30 mm, between 500 µm and 25 mm, between 750 µm and 20 mm, between 1 mm and 10 mm, between 2 mm and 5 mm, or between 1 mm and 2 mm. In certain cases, the desired depth can be selected based on the known location of the chromophore of interest. For example, sebaceous glands (and the sebum contained therein) are known to generally be positioned at a known depth range beneath the surface of skin of between 1 mm and 2 mm.

In certain aspects, the phototreating can be applied at a phototreating absorbed average power density or absorbed average irradiance of between 1 W/cm² and 100 W/cm², including but not limited to, a phototreating absorbed average power density or absorbed average irradiance of between 2 W/cm² and 90 W/cm², between 3 W/cm² and 80 W/cm², between 4 W/cm² and 75 W/cm², between 5 W/cm² and 70 W/cm², between 6 W/cm² and 60 W/cm², between 7 W/cm² and 50 W/cm², between 8 W/cm² and 40 W/cm², between 9 W/cm² and 30 W/cm², between 10 W/cm² and 25 W/cm², between 15 W/cm² and 20 W/cm², between 20 W/cm² and 35 W/cm², between 25 W/cm² and 45 W/cm², between 30 W/cm² and 55 W/cm², between 35 W/cm² and 65 W/cm², or between 40 W/cm² and 85 W/cm². In certain aspects, the phototreating can be applied for a length of time of between 10 fs and 1 s, including but not limited to, a length of time of between 100 fs and 900 ms, between 1 ps and 800 ms, between 10 ps and 750 ms, between 100 ps and 700 ms, between 1 µs and 600 ms, between 10 µs and 500 ms, between 100 µs and 400 ms, between 1 ms and 300 ms, between 5 ms and 250 ms, between 10 ms and 200 ms, between 25 ms and 100 ms, between 50 ms and 75 ms, between 100 ms and 350 ms, between 200 ms and 450 ms, between 250 ms and 550 ms, or between 300 ms and 650 ms. In certain aspects, the phototreating can be applied for a single pulse or for multiple pulses. In the case of a single pulse, the time scale of the single pulse should be on the order of the relaxation time of the target chromophore. In the case of multiple pulses, the timing between pulses should be shorter than the relaxation time of the target chromophore.

It should be appreciated that the parameters for the phototreating will be dependent upon the chromophore of interest, the properties of the surrounding medium, and the properties of the thermal gradient 302.

In certain aspects, the methods described herein can be achieved using a single light source. In certain aspects, the methods described herein can be achieved without varying the power output of the single light source. However, even with a single light source and non-varying power output, the methods can still utilize a lower power application and/or shorter length of time for the preconditioning when compared with the phototreatment. One way to achieve this effect is by scanning the light across the target medium at a relatively fast speed to establish the preconditioning (the fast speed means a lower exposure for a given point), then scanning the light across the target medium at a relatively slow speed, which can include pausing over given points (the slow speed and/or pausing over given points means a higher exposure for the given points).

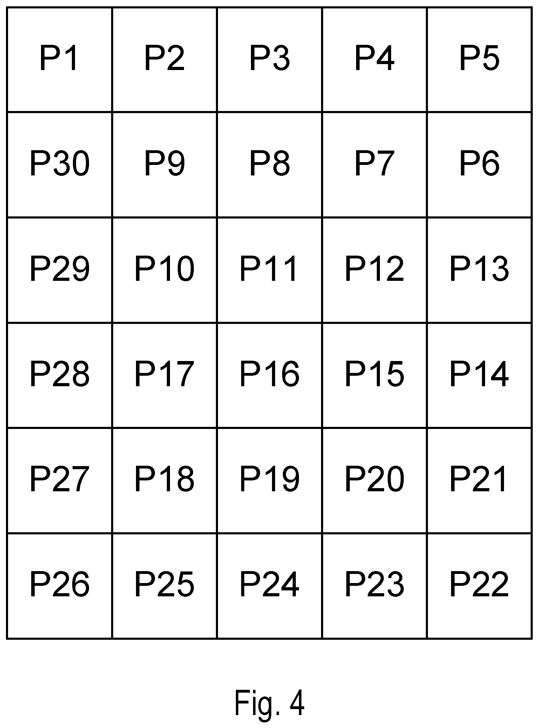

In certain aspects, the scanning can include raster scanning. Referring to Fig. 4, one potential scanning pattern is illustrated, with the sequential numbers identifying a sequence of positions for scanning the light (in other words, point P1 is illuminated first, followed by point P2, point P3, ..., etc. until point P30 is illuminated, after which point P1 is illuminated again). Note: the precooling can be applied to all of the points at the same time or can be scanned in a similar fashion.

A non-limiting example of a scanning protocol can include preconditioning scanning a 60 W laser in the pattern shown in Fig. 4 at a speed such that the light illuminates each position for 1/30th of a second, thus providing an effective power of 2 W of light to each position. This preconditioning scanning can be done for a length of time described herein, such as 20 seconds, thereby providing an effective 2 W preconditioning laser treatment for 20 seconds at each point. Subsequent to this preconditioning scanning, the non-limiting example of the scanning protocol can include phototreatment scanning the 60 W laser in the pattern shown in Fig. 4, pausing at each point for 0.2 seconds, thus providing a 60 W phototreatment for 0.2 seconds at each point. Again, this example is not intended to be limiting and many other scanning protocols are contemplated and would be apparent to a person having ordinary skill in the art in view of the present disclosure. This description of the preconditioning scanning should be understood as describing the concept of instantaneous power density (the power density of the laser, regardless of the scanning speed) and average power density (the power density experienced for a given position). The average power density drives bulk tissue heating and is balanced by the power density removal that results from the cooling effect of surrounding tissue.

The methods described herein can selectively target a chromophore of interest that is embedded in a surrounding medium. The chromophore of interest can be any chromophore described above. In certain aspects, the chromophore of interest can be sebum, melanin, hemoglobin, oxyhemoglobin, reduced hemoglobin, water, and the like. In certain aspects, the chromophore of interest can be sebum. The surrounding medium can be a tissue, including but not limited to, a layer of skin, such as an epidermis or a dermis, a subcutaneous tissue, a muscle tissue, a fat tissue, brain tissue, organ tissue, and the like; biological fluids, including but not limited to, blood, blood plasma, lymph, urine, bile, and the like; and the like.

In certain aspects, the chromophore of interest can have a selectivity to the phototreating when compared relatively to the surrounding medium. In certain aspects, the selectivity (as a ratio of optical absorption or the chromophore of interest relative to the surrounding medium) can be between 2 and 1, including but not limited to, a selectivity of between 1.95 and 1.1, between 1.9 and 1.2, between 1.85 and 1.3, between 1.8 and 1.4, between 1.75 and 1.5, between 1.7 and 1.6, between 1.6 and 1.05, between 1.5 and 1.15, or between 1.4 and 1.25 relative to the surrounding medium. It should be appreciated that the heat capacity of the chromophore of interest and the surrounding medium can impact the resulting temperature that is achieved by the selectivity. For example, with a chromophore of interest having a selectivity of 1 relative to the surrounding medium and a heat capacity that is higher than the surrounding medium, a higher temperature in the chromophore of interest can be achieved.

In certain aspects, the methods described herein can be useful for targeting red hair for photothermolytic removal. In other aspects, the methods described herein can be useful for speeding recovery of contusions or bruises.

Aspects of the disclosure described elsewhere herein with respect to the system 10 are applicable to the methods, unless the context clearly dictates otherwise. For example, if a given wavelength or pulse width is described with respect to the system 10, then the method should be interpreting as utilizing that given wavelength or pulse width.

### EXAMPLES

### Example 1.

A custom-built laser operating at a wavelength of 1726 nm and having a 5 mm spot size with a substantially uniform intensity distribution was used for this Example and the other Examples that follow.

A preheating application of 1 W to a target medium of porcine skin tissue for varying periods of time to determine a damage threshold. Application of the 1 W of 1726 nm light for 15-30 seconds produced no damage. Application of the same light for 40 seconds caused damage to a volume 2 mm wide and 1.5 mm deep. Application of the same light for 60 seconds caused damage to a volume 3.5 mm wide and 2 mm deep.

### Example 2.

The laser of Example 1 was utilized in human subject testing to determine pain thresholds for various intensities of single pulses of light. The pulses of light had a pulse width of 100 ms. No surface cooling was applied. Application of pulses of light with power of 25-40 W showed only mild symptoms in the subject. Application of pulses of light with power of 45 W was uncomfortable for the subject. Application of pulses of light with power of 55 W was painful for the subject and accompanied by skin blanching in the subject.

### Example 3.

The laser of Example 1 was utilized to determine a damage threshold for a single pulse, using an *ex vivo* porcine skin sample. A single 100 ms pulse of 45-55 W light at 1726 nm without Zimmer cooling produced non-selective damage. A single 100 ms pulse of 45-55 W light at 1726 nm *with* Zimmer cooling produced no damage. A preheating 1726 nm light of 1 W power was applied with a -5.5 °C Zimmer cooling for 15 seconds to produce a temperature gradient in the porcine skin sample. Following the preheating, a single 200 ms pulse of 30 W light at 1726 nm (with Zimmer cooling still active, though similar results would be expected if Zimmer cooling were discontinued immediately prior to application of the single pulse) was applied, resulting in skin damage 1.5 mm deep.

### Example 4.

The laser of Example 1 and the Zimmer cooling device of Example 3 were utilized in this Example. A precooling -5.5 °C Zimmer cooling was applied for 15-20 seconds, followed by varying numbers of 100 ms pulses of 15 W light at 1726 nm.

In all cases in this and following Examples, images were taken with a purple staining where purple color represented areas of tissue that were not damaged and white color represented areas of tissue that were damaged.

The application of 3 pulses resulted in a partially damaged area that extended to a depth in the skin sample that was not completely free of purple staining across a width of the sample. The depth of the partially-damaged area extended up to a thickness of the sample that was not damaged at the epidermis which extended across a width of the sample. The partially-damaged area was approximately uniform in damage across the width of the sample, and the damage area did not feature complete removal of the purple stain. The partially-damaged area featured dispersed areas that were left undamaged, and resulted in non-selective damage of the sample.

In another application of 3 pulses to the sample, similar results were achieved in that the epidermis was spared and the sample featured non-selective damage below the epidermis. A small semi-spherical region in the dermis of the sample located at the surface of the sample showed damage which extended from the surface to a minor depth within the sample.

The application of 7 pulses resulted in the epidermis being spared and the sample featured non-selective damage below the epidermis. A non-selective damage area in the dermis of the sample was centrally located on the sample and extended from the surface of the sample in a semi-spherical shape towards but not into the epidermis. The damage area featured dispersed areas that were left undamaged, and resulted in non-selective damage of the sample. A partially-damaged area surrounded the semi-spherical damaged area and featured dispersed areas that were left undamaged, and resulted in non-selective damage of the sample below the epidermis.

The application of 8 pulses resulted in a similar result to the application of 3 pulses. The depth of the partially-damaged area extended up to a thickness of the sample that was not damaged at the epidermis which extended across a width of the sample. The partially-damaged area was approximately uniform in damage across the width of the sample, and the damage area did not feature complete removal of the purple stain. The partially-damaged area featured dispersed areas that were left undamaged, and resulted in non-selective damage of the sample.

The resulting damage in Example 4 was inconsistent with some samples showing minimal to no damage and others showing non-selective damage.

### Example 5.

The laser of Example 1 and the Zimmer cooling device of Example 3 were utilized in this Example. Various length of -5.5 °C Zimmer precooling and various numbers of 100 ms pulses of 30 W light at 1726 nm were applied to determine minimum damage thresholds.

Applying Zimmer precooling for 1 second prior to applying 1 pulse resulted in non-damage of the tissue sample. The purple stain remained throughout the sample and demonstrated non-damage throughout the sample.

Applying Zimmer precooling for 1 second prior to applying 3 pulses resulted in non-selective damage of the tissue sample that featured a partially-damaged area of the sample between the surface of the sample and the epidermis of the sample. The partially-damaged area resulted in a faded spherical area of the sample that was partially-damaged at its center and faded into the non-damaged region that surrounded the partially-damaged area. This partially-damaged area resulted in a non-selective damage area of the sample.

Applying Zimmer precooling for 1 second prior to applying 6 pulses resulted in the epidermis remaining non-damaged while a non-selective damage area extended from a surface of the sample to the epidermis of the sample. The non-selective damage area in the dermis of the sample featured a damage area free of purple stain that extended from at a first area of the surface of the sample to a second area on the surface of the sample. The damage area extended from the first area on the surface to the second area on the surface in an approximately U-shaped manner such that an intermediate area of the surface between the first area and the second area was left undamaged. The U-shaped damage area featured a thickness near the thickness of the sample between the epidermis and the surface of the sample that tapered near the first area and the second area of the surface. This resulted in non-selective damage of the tissue sample.

Applying Zimmer precooling for 10 to 30 second prior to applying 6 pulses resulted in either non-damage of the sample or non-selective damage of the sample. The non-selective damage of the sample resulted in an elliptical-shaped damage area near the surface of the sample that was surrounded by a non-damaged area between the surface and the epidermis. In each of these samples, the epidermis was also non-damaged.

The results of Example 5 showed inconsistent results with some settings showing non-damage and others showing non-selective damage.

### Example 6.

The laser of Example 1 and the Zimmer cooling device of Example 3 were utilized in this Example.

A thermal gradient was established in *ex vivo* porcine skin by applying Zimmer precooling for 20 seconds at -5.5 °C in combination with preheating with 1726 nm light at various powers. After the establishment of the thermal gradient, a single 100 ms pulse of 35 W light at 1726 nm was applied.

Applying 6.5 W preheating resulted in non-selective damage of the majority of the tissue sample such that the surface of the sample and a surface of the epidermis positioned away from the surface of the sample remain undamaged while the rest of the sample is non-selectively damaged and free of the previously applied purple stain.

Applying a 2.5 W preheating resulted in little to no damage of the tissue sample. The epidermis was spared and between the surface of the sample and the epidermis little to no damage was shown.

Applying a 3.0 W preheating resulted in deep dermis damage that spares the epidermis from damage. The deep dermis damage had an area that was spherical in shape and extended between the epidermis and the surface of the dermis of the sample. This resulted in a semi-selective damage area of the sample while having a partially damaged area surrounding the damage area between the epidermis and the surface of the dermis of the sample.

The results of Example 6 showed non-selective damage for the 6.5 W preheating, little to no damage for the 2.5 W preheating, and deep dermis damage that spares the epidermis for the 3.0 W preheating.

### Example 7.

The laser of Example 1 and the Zimmer cooling device of Example 3 were utilized in this Example.

A thermal gradient was established by applying Zimmer precooling for 20 seconds at -5.5 °C in combination with preheating for the same 20 seconds with 1726 nm light at 2.5 W. After establishment of the thermal gradient, a single 100 ms pulse of 35 W light at 1726 nm was applied. Following the pulse, a Zimmer post-cooling of 20 seconds at - 5.5 °C was applied. Selective damage was achieved, including damage to a sebaceous gland, and the epidermis was spared damage. The selective damage was spherical in shape and lacked a surrounding area of damaged or partially-damaged sample resulting in the selective damage.

### Example 8.

The laser of Example 1 and the Zimmer cooling device of Example 3 were utilized in this Example.

1726 nm light at various powers was applied to the skin of a healthy human volunteer with and without Zimmer cooling at -5.5 °C to determine a pain threshold. When applying 2.0 W of 1726 nm light, the pain threshold was reached in 7 second without Zimmer cooling and 8 seconds with Zimmer cooling. When applying 1.5 W of 1726 nm light, the pain threshold was reached in 5-8 seconds without Zimmer cooling and 20 seconds with Zimmer cooling. When applying 1.0 W of 1726 nm light, the pain threshold was reached in 15 seconds without Zimmer cooling and 40-57 seconds with Zimmer cooling.

### Example 9.

The laser of Example 1 and the Zimmer cooling device of Example 3 were utilized in this Example.

A thermal gradient was established in the skin of a healthy human volunteer by applying Zimmer precooling for 20 seconds at -6 °C in combination with preheating with 1726 nm light at 1.5 W. After the establishment of the thermal gradient, a single 150 ms pulse of varying power light at 1726 nm was applied. For a single 150 ms pulse of 15-30 W light at 1726 nm, the pain was mild to moderate. For a single 150 ms pulse of 35-50 W light at 1726 nm, the pain was moderate to sharp. A clinical papule was observed with the 45 W pulse.

### Example 10.

The laser of Example 1 and the Zimmer cooling device of Example 3 were utilized in this Example.

A thermal gradient was established in *ex vivo* porcine skin by applying preheating with 1726 nm light at 1.5 W for 20 seconds, with and without Zimmer precooling for 20 seconds at -5.5 °C. After the establishment of the thermal gradient, a single 150 ms pulse of 50 W light at 1726 nm was applied. The absence of Zimmer precooling resulted in damage that occurred 1mm into the sample while sparing the epidermis. The damage area featured an elliptical shape oriented horizontally across the sample at a depth of 1mm into the sample.

The presence of Zimmer precooling resulted in, similar to the absence of Zimmer precooling, the damage was 1 mm deep and the epidermis was spared. The damage area was slightly smaller in the presence of Zimmer precooling, thus resulting in a slightly more selective damage area than without Zimmer precooling.

### Example 11.

The laser of Example 1 and the Zimmer cooling device of Example 3 were utilized in this Example.

A thermal gradient was established in *ex vivo* porcine skin by applying preheating with 1726 nm light at varying powers for 20 seconds. Preheating at 4.0 W resulted in non-selective damage of the majority of the tissue sample such that the surface of the sample and a surface of the epidermis positioned away from the surface of the sample remain undamaged while the rest of the sample is non-selectively damaged and free of the previously applied purple stain.

When preheating was applied at 2.0 W, a thermal gradient was also established in *ex vivo* porcine skin by applying preheating with 1726 nm light at 1.5 W for 40 seconds. This resulted in non-selective dermal damage of the sample that was cylindrical in shape and extended between the epidermis and a surface of the dermis of the sample. The damage was surrounded by an area of the dermis that was not damaged and left the epidermis undamaged.

When preheating was applied at 1.5 W for 40 seconds r similarly resulted in non-selective dermal damage of the sample that was cylindrical in shape and positioned between the epidermis and a surface of the dermis of the sample. The damage was surrounded by an area of the dermis that was not damaged and left the epidermis undamaged or partially-damaged.

### Example 12.

The laser of Example 1 and the Zimmer cooling device of Example 3 were utilized in this Example. The results in this Example were achieved using the experimental setup shown in Fig. 13. The skin sample was placed such that a cut edge of the skin is positioned half way through the laser beam. The heating plate was maintained at 37 °C in order to simulate body temperature. The Zimmer cooling device was maintained at -5 °C.

In addition to the Zimmer precooling, a preheating was applied with 1726 nm light at 0.5 W for 20 seconds, followed by a single pulse of 1726 nm light at power ranging from 3 W to 30 W and pulse widths ranging from 100 ms to 175 ms. The maximum temperature measured at the epidermis was 18 °C. The maximum temperature measured at the dermis was 53 °C achieved by a 150 ms pulse at 30 W. Temperatures of 5-17 °C at the epidermis and 44 °C at the dermis were achieved by a 150 ms pulse at 20 W. Temperatures of 9-11 °C at the epidermis and 37 °C at the dermis were achieved by a 150 ms pulse at 150 ms.

In addition to the Zimmer precooling, a preheating was applied with 1726 nm light at 0.5 W for 20 seconds, followed by varying numbers of pulses of 1726 nm light at a power of 10 W and pulse widths of 150 ms. When 3 pulses were applied, temperatures of 3-10 °C at the epidermis and 28 °C at the dermis were achieved. When 6 pulses were applied, temperatures of 6 °C at the epidermis and 37 °C at the dermis were achieved. When 9 pulses were applied, temperatures of 23 °C at the epidermis and 37 °C at the dermis were achieved. When 15 pulses were applied, with a 700 ms delay between pulses, temperatures of 32 °C at the epidermis and 57 °C at the dermis were achieved. In the 15 pulse application, the temperature of the dermis was measured after each pulse, and the results are as follows (nomenclature is pulse number = temperature: 1=29 °C; 2=36 °C; 3=40 °C; 4=43 °C; 5=46 °C; 6=48 °C; 7=49.7 °C; 8=51 °C; 9=52.3 °C; 10=53.2 °C; 11=53.6 °C; 12 =55 °C; 13 = 55.7 °C; 14 =57 °C; and 15 =57 °C.

### Example 13.

The laser of Example 1 and the Zimmer cooling device of Example 3 were utilized in this Example. The experimental setup of Example 12 was used to acquire measurements.

In addition to the Zimmer precooling, a preheating was applied with 1726 nm light at 0.5 W for 20 seconds, followed by single or multiple 150 ms pulses of 1726 nm light at 10 W. With the Zimmer precooling alone (i.e., without the preheating or the 150 ms pulses at 10 W), the temperature of the dermis was 15 °C. With the preheating alone (i.e., without the 150 ms pulses at 10 W), the temperature of the dermis was 24 °C. When 1 pulse was applied with the Zimmer precooling but without the preheating, the temperature of the dermis was 32 °C. When 3 pulses were applied with the Zimmer precooling and the preheating, the temperature of the dermis was 29 °C. When 4 pulses were applied with the Zimmer precooling and the preheating, the temperature of the dermis was 40 °C. When 5 pulses were applied with the Zimmer precooling and the preheating, the temperature of the dermis was 54-56 °C. When 8 pulses were applied with the Zimmer precooling and the preheating, the temperature of the dermis was 48 °C. Nitro blue tetrazolium chloride (NBTZ) staining was applied to all samples and no epidermis damage was found.

### Example 14.

The laser of Example 1 and the Zimmer cooling device of Example 3 were utilized in this Example. A control sample of human cadaver skin has an epidermis (Ep), a hair follicle (HF), and a sebaceous gland (SG). The human cadaver skin was positioned between the cooling window of the Zimmer cooling device and a heating plate maintained at 37 °C in order to simulate body temperature.

The human cadaver skin sample was treated with two 150 ms pulses of 35 W light with a pulse delay of 1000 ms and no pre-heating, which resulted in selective damage of portions of the sebaceous glad. Some non-selective damage was shown around the sebaceous gland.

The human cadaver skin sample was treated with two 150 ms pulses of 40 W light with a pulse delay of 1000 ms and no pre-heating, which resulted in selective damage of portions of the sebaceous glad. Some non-selective damage was shown around the sebaceous gland, specifically between the sebaceous gland and the epidermis.

The human cadaver skin sample was treated with two 150 ms pulses of 35 W light with a pulse delay of 1000 ms and preheating at 0.5 W for 20 seconds, which resulted in selective damage of portions of the sebaceous glad. A minimal amount of non-selective damage was shown around the sebaceous gland.

### THE GENERAL HOSPITAL CORPORATION

As will be recognized, certain embodiments of the disclosures described herein can be embodied within a form that does not provide all of the features and benefits set forth herein, as some features can be used or practiced separately from others. The scope of certain disclosures disclosed herein is indicated by the appended claims rather than by the foregoing description. The invention is defined in the appended claims.

## Claims

1. A system for controlled thermal treatment of a target, which contains a chromophore of interest, embedded in a surrounding medium, the system comprising:
a preconditioning cooling device (22) configured for applying cooling to a surface of the surrounding medium, and a preconditioning light source (24) configured to transmit light into the surrounding medium, the target, and the chromophore of interest;
a phototreatment light source (30) configured to transmit light into the surrounding medium, the target, and the chromophore of interest;
a processor in communication with the preconditioning cooling device (22), the preconditioning light source (24) and the phototreatment light source (30) and configured to control the preconditioning cooling device (22), the preconditioning light source (24) and the phototreatment light source (30); and
a memory having stored thereon instructions that, when executed by the processor, cause the processor to execute a preconditioning and a phototreatment,
wherein the processor, during the preconditioning, controls the preconditioning cooling device (22) and the preconditioning light source (24) to establish a thermal gradient in the surrounding medium, the thermal gradient having a peak temperature within a desired depth beneath a surface of the surrounding medium, wherein the desired depth is selected based on a known depth of the target and the chromophore of interest,
wherein the processor, during the phototreatment, controls the phototreatment light source (30),
wherein the preconditioning light source (24) and the phototreatment light source (30) are a single light source,
wherein the single light source is a laser,
wherein the single light source is configured to provide pulsed light during the preconditioning and the phototreatment,
wherein the single light source is configured to provide light having a wavelength of between 1600 nm and 1800 nm.

2. The system of claim 1, wherein the single light source is configured to provide light having a wavelength that is tuned to a peak absorption of the chromophore of interest.

3. The system of claim 2, wherein the preconditioning selectively heats the chromophore of interest to a higher temperature than the surrounding medium.

4. The system of any one of the preceding claims, wherein the processor, during the preconditioning, controls the preconditioning cooling device (22) and the preconditioning light source (24) to simultaneously apply cooling from the preconditioning cooling device (22) to the surface of the surrounding medium and transmit light from the preconditioning light source (24) into the surrounding medium, and wherein the processor, during the phototreatment, optionally controls the preconditioning cooling device (22).

5. The system of any one of the preceding claims, wherein the desired depth is between 1 µm and 100 mm.

6. The system of any one the preceding claims, wherein the chromophore of interest is sebum and the target is the sebaceous gland.

7. The system of any one of the preceding claims, wherein the surrounding medium is located on or in a subject.

8. The system of any one of the preceding claims, wherein the single light source has a non-varying power output.

9. The system of any one of the preceding claims, wherein the processor executes the preconditioning for a preheating length of time of between 1 second and 5 minutes or the processor executes the phototreatment for a phototreatment length of time of between 10 femtoseconds and 1 second.

10. A cosmetic method of controlled thermal treatment of a target, which contains a chromophore of interest, embedded in a surrounding medium, wherein the chromophore of interest is sebum, the method comprising the following steps:
a) establishing, using a preconditioning cooling device (22) and a preconditioning light source (24), a thermal gradient in the surrounding medium by applying cooling to a surface of the surrounding medium and transmitting light into the surrounding medium, the target, and the chromophore of interest, the thermal gradient having a peak temperature within a desired depth beneath a surface of the surrounding medium, wherein the desired depth is selected based on a known depth of the target and the chromophore of interest; and
b) selectively heating, using a photothermal light source (30) and subsequent to step a), the chromophore of interest by an amount sufficient to exceed a damage threshold of the target at the desired depth while not exceeding a damage threshold of the surrounding medium at the desired depth,
wherein the preconditioning light source (24) and the photothermal light source (30) are a single light source wherein the single light source is a laser configured to provide pulsed light having a wavelength of between 1600 nm and 1800 nm.

11. The cosmetic method of claim 10, wherein step a) or step b) uses light from the single light source having a wavelength that is tuned to a peak absorption of the chromophore of interest, and wherein step a) optionally selectively heats the chromophore of interest to a higher temperature than the surrounding medium.

## Patentansprüche

1. System zur kontrollierten Wärmebehandlung eines Ziels, das einen interessierenden Chromophor, eingebettet in ein umgebendes Medium, enthält, wobei das System umfasst:
eine Vorkonditionierungskühlvorrichtung (22), die dazu eingerichtet ist, eine Kühlung auf eine Oberfläche des umgebenden Mediums anzuwenden, und eine Vorkonditionierungslichtquelle (24), die dazu eingerichtet ist, Licht in das umgebende Medium, das Ziel und den interessierenden Chromophor zu senden;
eine Lichtbehandlungslichtquelle (30), die dazu eingerichtet ist, Licht in das umgebende Medium, das Ziel und den interessierenden Chromophor zu senden;
einen Prozessor in Kommunikation mit der Vorkonditionierungskühlvorrichtung (22), der Vorkonditionierungslichtquelle (24) und der Lichtbehandlungslichtquelle (30) und dazu eingerichtet, die Vorkonditionierungskühlvorrichtung (22), die Vorkonditionierungslichtquelle (24) und die Lichtbehandlungslichtquelle (30) zu steuern; und
einen Speicher, in dem Instruktionen gespeichert sind, die, wenn sie durch den Prozessor ausgeführt werden, den Prozessor veranlassen, eine Vorkonditionierung und eine Lichtbehandlung durchzuführen,
wobei der Prozessor, während der Vorkonditionierung, die Vorkonditionierungskühlvorrichtung (22) und die Vorkonditionierungslichtquelle (24) steuert, einen Temperaturgradienten in dem umgebenden Medium zu erzeugen, wobei der thermische Gradient eine Spitzentemperatur innerhalb einer gewünschten Tiefe unterhalb einer Oberfläche des umgebenden Mediums aufweist, wobei die gewünschte Tiefe auf der Grundlage einer bekannten Tiefe des Ziels und des interessierenden Chromophors ausgewählt wird,
wobei der Prozessor, während der Lichtbehandlung, die Lichtbehandlungslichtquelle (30) steuert,
wobei die Vorkonditionierungslichtquelle (24) und die Lichtbehandlungslichtquelle (30) eine einzige Lichtquelle sind,
wobei die einzige Lichtquelle ein Laser ist,
wobei die einzige Lichtquelle dazu eingerichtet ist, während der Vorkonditionierung und der Lichtbehandlung gepulstes Licht bereitzustellen,
wobei die einzige Lichtquelle dazu eingerichtet ist, Licht mit einer Wellenlänge zwischen 1600 nm und 1800 nm bereitzustellen.

2. System nach Anspruch 1, wobei die einzige Lichtquelle dazu eingerichtet ist, Licht mit einer Wellenlänge bereitzustellen, die auf eine Spitzenabsorption des interessierenden Chromophors abgestimmt ist.

3. System nach Anspruch 2, wobei die Vorkonditionierung den interessierenden Chromophor selektiv auf eine höhere Temperatur als das umgebende Medium erwärmt.

4. System nach einem der vorangehenden Ansprüche, wobei der Prozessor, während der Vorkonditionierung, die Vorkonditionierungskühlvorrichtung (22) und die Vorkonditionierungslichtquelle (24) so steuert, dass gleichzeitig eine Kühlung von der Vorkonditionierungskühlvorrichtung (22) auf die Oberfläche des umgebenden Mediums angewendet wird und Licht von der Vorkonditionierungslichtquelle (24) in das umgebende Medium gesendet wird, und wobei der Prozessor, während der Lichtbehandlung, optional die Vorkonditionierungskühlvorrichtung (22) steuert.

5. System nach einem der vorangehenden Ansprüche, wobei die gewünschte Tiefe zwischen 1 µm und 100 mm liegt.

6. System nach einem der vorangehenden Ansprüche, wobei der interessierende Chromophor Talg ist und das Ziel die Talgdrüse ist.

7. System nach einem der vorangehenden Ansprüche, wobei sich das umgebende Medium auf oder in einer Person befindet.

8. System nach einem der vorangehenden Ansprüche, wobei die einzige Lichtquelle eine unveränderliche Leistungsabgabe aufweist.

9. System nach einem der vorangehenden Ansprüche, wobei der Prozessor die Vorkonditionierung über eine Vorwärmzeitdauer zwischen 1 Sekunde und 5 Minuten ausführt oder der Prozessor die Lichtbehandlung über eine Lichtbehandlungszeitdauer zwischen 10 Femtosekunden und 1 Sekunde ausführt.

10. Kosmetisches Verfahren zur kontrollierten Wärmebehandlung eines Ziels, das einen interessierenden Chromophor, eingebettet in ein umgebendes Medium, enthält, wobei der interessierende Chromophor Talg ist, wobei das Verfahren die folgenden Schritte umfasst:
a) Erzeugen, unter Verwendung einer Vorkonditionierungskühlvorrichtung (22) und einer Vorkonditionierungslichtquelle (24), eines thermischen Gradienten in dem umgebenden Medium durch Anwenden einer Kühlung auf eine Oberfläche des umgebenden Mediums und Senden von Licht in das umgebende Medium, das Ziel und den interessierenden Chromophor, wobei der thermische Gradient eine Spitzentemperatur innerhalb einer gewünschten Tiefe unterhalb einer Oberfläche des umgebenden Mediums aufweist, wobei die gewünschte Tiefe auf der Grundlage einer bekannten Tiefe des Ziels und des interessierenden Chromophors ausgewählt wird; und
b) selektives Erwärmen, unter Verwendung einer photothermischen Lichtquelle (30) und im Anschluss an Schritt a), des interessierenden Chromophors um einen Betrag, der ausreicht, um eine Schädigungsschwelle des Ziels in der gewünschten Tiefe zu überschreiten, ohne eine Schädigungsschwelle des umgebenden Mediums in der gewünschten Tiefe zu überschreiten,
wobei die Vorkonditionierungslichtquelle (24) und die photothermische Lichtquelle (30) eine einzige Lichtquelle sind, wobei die einzige Lichtquelle ein Laser ist, der dazu eingerichtet ist, gepulstes Licht mit einer Wellenlänge zwischen 1600 nm und 1800 nm bereitzustellen.

11. Kosmetisches Verfahren nach Anspruch 10, wobei Schritt a) oder Schritt b) Licht von der einzigen Lichtquelle mit einer Wellenlänge verwendet, die auf eine Spitzenabsorption des interessierenden Chromophors abgestimmt ist, und wobei Schritt a) optional den interessierenden Chromophor selektiv auf eine höhere Temperatur als das umgebende Medium erwärmt.

## Revendications

1. Système de traitement thermique contrôlé d'une cible, qui contient un chromophore d'intérêt, enfoncé dans un milieu environnant, le système comprenant :
un dispositif de refroidissement de préconditionnement (22) conçu pour appliquer un refroidissement sur une surface du milieu environnant, et une source de lumière de préconditionnement (24) conçue pour émettre de la lumière dans le milieu environnant, la cible et le chromophore d'intérêt ;
une source de lumière de photo-traitement (30) conçue pour émettre de la lumière dans le milieu environnant, la cible et le chromophore d'intérêt ;
un processeur en communication avec le dispositif de refroidissement de préconditionnement (22), la source de lumière de préconditionnement (24) et la source de lumière de photo-traitement (30) et conçu pour commander le dispositif de refroidissement de préconditionnement (22), la source de lumière de préconditionnement (24) et la source de lumière de photo-traitement (30) ; et
une mémoire sur laquelle sont enregistrées des instructions qui, lorsqu'elles sont exécutées par le processeur, amènent le processeur à exécuter un préconditionnement et un photo-traitement,
dans lequel le processeur, pendant le préconditionnement, commande le dispositif de refroidissement de préconditionnement (22) et la source de lumière de préconditionnement (24) pour établir un gradient thermique dans le milieu environnant, le gradient thermique présentant une température maximale l'intérieur d'une profondeur souhaitée sous la surface du milieu environnant, la profondeur souhaitée étant choisie sur la base d'une profondeur connue de la cible et du chromophore d'intérêt,
dans lequel le processeur, pendant le photo-traitement, commande la source de lumière de photo-traitement (30),
dans lequel la source de lumière de préconditionnement (24) et la source de lumière de photo-traitement (30) sont une unique source de lumière,
dans lequel l'unique source de lumière est un laser,
dans lequel l'unique source de lumière est conçue pour fournir de la lumière pulsée pendant le préconditionnement et le photo-traitement,
dans lequel l'unique source de lumière est conçue pour fournir de la lumière présentant une longueur d'onde comprise entre 1600 nm et 1800 nm.

2. Système selon la revendication 1, dans lequel l'unique source de lumière est conçue pour fournir de la lumière présentant une longueur d'onde qui est réglée sur une absorption maximale du chromophore d'intérêt.

3. Système selon la revendication 2, dans lequel le préconditionnement chauffe, de manière sélective, le chromophore d'intérêt à une température plus élevée que le milieu environnant.

4. Système selon l'une quelconque des revendications précédentes, dans lequel le processeur, pendant le préconditionnement, commande le dispositif de refroidissement de préconditionnement (22) et la source de lumière de préconditionnement (24) pour, simultanément, appliquer un refroidissement émanant du dispositif de refroidissement de préconditionnement (22) sur la surface du milieu environnant et émettre de la lumière émanant de la source de lumière de préconditionnement (24) dans le milieu environnant, et dans lequel le processeur, pendant le photo-traitement, commande en option le dispositif de refroidissement de préconditionnement (22).

5. Système selon l'une quelconque des revendications précédentes, dans lequel la profondeur souhaitée est comprise entre 1 µm et 100 mm.

6. Système selon l'une quelconque des revendications précédentes, dans lequel le chromophore d'intérêt est le sébum et la cible est la glande sébacée.

7. Système selon l'une quelconque des revendications précédentes, dans lequel le milieu environnant est situé sur ou dans un sujet.

8. Système selon l'une quelconque des revendications précédentes, dans lequel l'unique source de lumière présente une puissance de sortie non variable.

9. Système selon l'une quelconque des revendications précédentes, dans lequel le processeur exécute le préconditionnement pour une durée de préchauffage comprise entre 1 seconde et 5 minutes ou le processeur exécute le photo-traitement pour une durée de photo-traitement comprise entre 10 femtosecondes et 1 seconde.

10. Procédé cosmétique de traitement thermique contrôlé d'une cible, qui contient un chromophore d'intérêt, enfoncé dans un milieu environnant, le chromophore d'intérêt étant le sébum, le procédé comprenant les étapes suivantes :
a) établissement, au moyen d'un dispositif de refroidissement de préconditionnement (22) et d'une source de lumière de préconditionnement (24), d'un gradient thermique dans le milieu environnant, en appliquant un refroidissement sur une surface du milieu environnant et en émettant de la lumière dans le milieu environnant, la cible et le chromophore d'intérêt, le gradient thermique présentant une température maximale à l'intérieur d'une profondeur souhaitée sous une surface du milieu environnant, la profondeur souhaitée étant choisie sur la base d'une profondeur connue de la cible et du chromophore d'intérêt ; et
b) de manière sélective, chauffage, au moyen d'une source de lumière photothermique (30) et après l'étape a), du chromophore d'intérêt à un degré suffisant pour dépasser un seuil d'endommagement de la cible à la profondeur souhaitée tout en ne dépassant pas un seuil d'endommagement du milieu environnant à la profondeur souhaitée,
dans lequel la source de lumière de préconditionnement (24) et la source de lumière photothermique (30) sont une unique source de lumière, l'unique source de lumière étant un laser conçu pour fournir de la lumière pulsée présentant une longueur d'onde comprise entre 1600 nm et 1800 nm.

11. Procédé cosmétique selon la revendication 10, dans lequel l'étape a) ou l'étape b) utilise de la lumière émanant de l'unique source de lumière présentant une longueur d'onde qui est réglée sur une absorption maximale du chromophore d'intérêt, et dans lequel l'étape a) en option, chauffe de manière sélective le chromophore d'intérêt à une température plus élevée que le milieu environnant.
